Europäisches Patentamt

European Patent Office

Office européen des brevets

(19)

(11) Veröffentlichungsnummer: **0 234 366**
**B1**

(12) **EUROPÄISCHE PATENTSCHRIFT**

(45) Veröffentlichungstag der Patentschrift:
13.06.90

(21) Anmeldenummer: 87101655.6

(22) Anmeldetag: 06.02.87

(51) Int. Cl.⁵: **A61B 17/22**

(54) Ankoppelkörper für eine Stosswellen-Therapieeinrichtung.

(30) Priorität: 19.02.86 DE 3605277

(43) Veröffentlichungstag der Anmeldung:
02.09.87 Patentblatt 87/36

(45) Bekanntmachung des Hinweises auf die Patenterteilung:
13.06.90 Patentblatt 90/24

(84) Benannte Vertragsstaaten:
DE FR GB NL

(56) Entgegenhaltungen:
DE-A- 2 913 251
DE-A- 3 312 014
FR-A- 2 552 611
GB-A- 2 003 701
GB-A- 2 102 657
US-A- 4 277 367

(73) Patentinhaber: Siemens Aktiengesellschaft,
Wittelsbacherplatz 2, D-8000 München 2(DE)

(72) Erfinder: Reichenberg, Helmut, Dr. Dipl.-Phys.,
Begonienstrasse 28, D-8501 Eckental(DE)
Erfinder: Naser, Georg, Dipl.-Ing. (FH), Karlstrasse 10,
D-8502 Zirndorf(DE)
Erfinder: Jahn, Helmut, Dipl.-Ing. (FH), Am
Eichengarten 11, D-8520 Erlangen-Buckenhof(DE)

ACTORUM AG

## Beschreibung

Die Erfindung betrifft einen Ankoppelkörper für die Übertragung von Stoßwellen von einer Stoßwellenquelle zu einem zu behandelnden Patienten.

Auf dem Gebiet der medizinischen Diagnosegeräte, die mit Ultraschall arbeiten, ist es bekannt, den Ultraschall-Applikator mittels einer Ankoppelpaste an den zu untersuchenden Patienten anzukoppeln. Eine solche Paste hat den Nachteil, daß beim Abnehmen und Wiederansetzen des Applikators sich an der Grenzfläche Applikator/Patient Lufteinschlüsse ergeben können, die zu Störungen im Ultraschallbild führen. Auch ist es auf diesem Arbeitsgebiet bekannt, zur diagnostischen Untersuchung von Patienten Vorlaufstrecken zu verwenden, die in ihrer Schallgeschwindigkeit dem Körpergewebe angepaßt sind. Bei diesen Vorlaufstrecken handelt es sich i.a. um Behälter (Beutel, Säcke o.dgl.), die mit einer Flüssigkeit, wie z.B. Wasser, gefüllt sind. Der Behälter wird in der Regel mit einer Ankoppelpaste einerseits am Ultraschall-Schwingungserreger und andererseits am Patienten angekoppelt. Auf diese Weise lassen sich störende Reflexionen an den Grenzflächen des Ultraschall-Applikators und des Untersuchungsobjekts vermeiden.

Die Erfindung bezieht sich nun nicht auf ein Ultraschall-Diagnosegerät, sondern auf eine Stoßwellen-Therapieeinrichtung, und zwar speziell auf eine Zertrümmerungseinrichtung für Konkremente im Körper eines Lebewesens. Eine solche Zertrümmerungseinrichtung ist beispielsweise in den DE-OS 3 328 039 und DE-OS 3 328 051 beschrieben. Auch bei solchen Zertrümmerungseinrichtungen hat man bisher zur Ankopplung und damit Übertragung von Stoßwellen auf dem Wege von der Stoßwellenquelle zu dem zu behandelnden Patienten eine Ankoppelpaste eingesetzt. Selbst wenn bei solchen Zertrümmerungseinrichtungen auf diesem Wege ein gummielastischer Körper (vgl. z.B. DE-OS 3 312 014), ein flüssigkeitsgefülltes Kissen (vgl. z.B. DE-OS 3 146 626) oder ein flüssigkeitsgefüller Faltenbalg (vgl. z.B. DE-OS 3 319 871) verwendet wurde, so mußte doch zur Ankopplung dieser elastischen Bauteile zumindest auf der Seite des Patienten eine fließfähige Ankoppelpaste benutzt werden. Der Einsatz einer solchen Ankoppelpaste erfordert aber eine eingehende Vorbehandlung des Patienten vor der eigentlichen therapeutischen Behandlung sowie eine sorgfältige Reinigung des Patienten und gegebenenfalls auch des Stoßwellengeräts nach der Benutzung.

Es wäre daher wünschenswert, wenn die Ankoppelpaste durch einen Ankoppelkörper ersetzt werden könnte, der einerseits eine akustisch sichere Ankopplung der Stoßwellen ohne Gaseinschlüsse an den Grenzflächen gewährleistet und der andererseits umfangreiche Reinigungsarbeiten nach der Behandlung überflüssig macht.

Aufgabe der vorliegenden Erfindung ist es somit, einen Ankoppelkörper für die Übertragung von Stoßwellen auf dem Wege von einer Stoßwellenquelle zu einem zu behandelnden Patienten anzugeben, der eine sichere, reproduzierbare Ankopplung sicherstellt, leicht handhabbar ist und ein sauberes Arbeiten gewährleistet.

Diese Aufgabe wird erfindungsgemäß dadurch gelöst, daß der Ankoppelkörper aus einem elastischen, formstabilen Material mit feuchten Außenflächen gebildet ist, daß er mit einer Einlage versehen ist, daß die Einlage über das elastische, formstabile Material übersteht und am überstehenden Teil Mittel zum Anfassen aufweist.

Aus der GB-A 2 003 701 ist ein Ankoppelkörper aus einem elastischen, formstabilen Material mit feuchten Außenflächen bekannt, der zur Ankopplung einer Ultraschallsonde an den Körper eines Patienten dient.

Bevorzugt kann das elastische, formstabile Material ein Hydro-Gel sein. Hydro-Gele sind Materialien, die als Matrix Wasser enthalten. Zu solchen Hydro-Gelen würde beispielsweise auch Gelatine zählen. Von besonderem Vorteil für den Einsatz bei der Übertragung von Stoßwellen ist jedoch ein Polyacrylamid-Gel. Ein solches Material ist sehr formstabil, d.h. leicht handhabbar, und leicht in eine bestimmte Form zu bringen. In der Benutzung paßt es sich leicht an die Konturen des Patientenkörpers einerseits und der Stoßwellen-Austrittsfläche andererseits an.

Das Polyacrylamid-Gel für den Ankoppelkörper wird vorzugsweise synthetisiert aus:

a) Acrylamid (als Monomer);
b) N,N'-Methylen-bis-Acrylamid (als Vernetzer);
c) Ammoniumperoxodisulfat (als Katalysator);
d) N,N,N',N'-Tetramethylethylen-Diamin (als Starter); und
e) Wasser (als Verdünnungsmittel).

Der Ansatz ist dünnflüssig wie Wasser. Man kann damit beliebige Formen des Ankoppelkörpers gießen. Nach erfolgter Polymerisation liegt ein glasklarer, weichelastischer und relativ reißfester Gel-Körper mit niedriger Dämpfung für die Stoßwellen und einer dem menschlichen Gewebe angepaßten Impedanz vor. Durch Abänderung des Monomer-Wasser-Verhältnisses lassen sich hartelastische bis dickflüssige Gele herstellen.

Polyacrylamid-Gele sind z.B. Copolymerisate von Acrylamid und Methylen-bis-acrylamid, deren Porenweite von der Abstufung zwischen der monomeren und dem quervernetzten Partner abhängig ist.

Polyacrylamid-Gele und Rezepturen zu ihrer Herstellung sind an sich bekannt. Sie eignen sich nach dem Stand der Technik als Molekularsiebe zum Trennen, Entsalzen und Konzentrieren von Substanzen mit hohem Molekulargewicht. Sie werden z.B. auch in der Gelchromatographie eingesetzt; ihre Herstellung kann nach bekannten Rezepturen aus käuflichen Ausgangsstoffen vorgenommen werden (H. Determann: «Gel-Chromatographie», Springer-Verlag, Berlin, Heidelberg, New York, insbesondere Seiten 19/21 und Seite 31). Ihre Eigenschaften sind eingehend untersucht worden (Spektrum der Wissenschaft, März 1981, Seiten 79 bis 93). Auch zur Elektrophorese werden Polyacrylamid-Gele verwendet, und zu diesem Zweck werden geeignete Reagenzien angeboten (Desaga Katalog, Fa. Desaga, Heidelberg, Seite 49 und 54).

Die Verwendung eines elastischen, formstabilen Materials mit feuchten Außenflächen als Ankoppelkörper gewährleistet eine akustisch sichere Ankopplung ohne Gaseinschlüsse in den Grenzflächen. Die Ankopplung ist reproduzierbar gut, d.h. bei Abnehmen und Wiederansetzen des Ankoppelkörpers gibt es infolge der Feuchtigkeit der Außenflächen keine störenden Lufteinschlüsse. Bei Verwendung eines optisch durchsichtigen Hydro-Gels können solche Lufteinschlüsse auch leicht mit dem Auge erkannt werden. Die Ankopplung ist sauber, d.h. es entstehen keine Verunreinigungen des Geräts und des Patienten bei der Benutzung. Infolge der Formstabilität des verwendeten Materials und infolge der eingeschlossenen Einlage ist der Ankoppelkörper leicht handhabbar, also z.B. leicht zu transportieren, aufzubewahren und zu justieren. Hydro-Gele sind im allgemeinen nicht teuer, und ihre Beseitigung nach der Benutzung ist nicht aufwendig.

Die Einlage ist bevorzugt ein nichtmetallisches Gewebe, z.B. eine Stoff-Gaze, das bei der Stoßwellenbehandlung keine Defokussierung oder sonstige Störung hervorruft. Sie steht randseitig etwas über und ist mit Mitteln zum Anfassen versehen. Diese Mittel können darin bestehen, daß das gesamte überstehende Teil ausreichend breit ist, um als Angrefffläche zu dienen. Stattdessen kann am überstehenden Teil auch eine Anfaßlasche vorgesehen sein.

Eine weitere bevorzugte Ausführungsform zeichnet sich dadurch aus, daß der Ankoppelkörper zugleich Ankoppelmedium und Vorlaufstrecke ist.

Der Ankoppelkörper kann in unterschiedlichen Dicken gefertigt werden und damit den Bedürfnissen in der Therapie angepaßt werden. Hydro-Gele und andere gleichartige Substanzen sind im allgemeinen nicht toxisch und gut hautverträglich. Handelt es sich um ein klares und durchsichtiges Material, was bevorzugt ist, so ist die betreffende Koppelstelle leicht überprüfbar.

Weitere vorteilhafte Ausgestaltungen sind in den Patentansprüchen gekennzeichnet. Die Erfindung wird im folgenden anhand von zehn Figuren näher erläutert. Es zeigen:

Fig. 1 eine erste Ausführungsform einer Stoßwellen-Therapieeinrichtung, bei der zwischen der Stoßwellen-Austrittsfläche und dem Patienten ein Ankoppelkörper aus einem elastischen, formstabilen Material mit feuchten Außenflächen angeordnet ist;
Fig. 2 eine zweite Ausführungsform einer Stoßwellen-Therapieeinrichtung mit konkaver Stoßwellen-Austrittsfläche, bei der ein ebensolcher Ankoppelkörper vorgesehen ist;
Fig. 3 einen Ankoppelkörper, der am Rand durch einen Schlauch eingefaßt ist;
Fig. 4 einen eingefaßten Ankoppelkörper zum Aufschieben auf das Endstück eines Stoßwellengeräts;
Fig. 5 einen scheibenförmigen Ankoppelkörper mit eingearbeiteter Gewebeeinlage;

Fig. 6 einen Aufblick auf einen im wesentlichen zylindrischen Ankoppelkörper mit Hüll-Verpackung;
Fig. 7 einen Schnitt durch einen eingefaßten Ankoppelkörper in einer topfförmigen Verpackung;
Fig. 8 einen Schnitt durch eine Stoßwellen-Therapieeinrichtung, bei der der Ankoppelkörper mittels seiner Gewebeeinlage an der Stoßwellenquelle befestigt ist;
Fig. 9 eine weitere Stoßwellen-Einrichtung, bei der der Ankoppelkörper mittels zweier Klemmzangen an der Stoßwellenquelle festgehalten ist; und
Fig. 10 eine der bei der Einrichtung nach Fig. 9 verwendeten Klemmzangen.

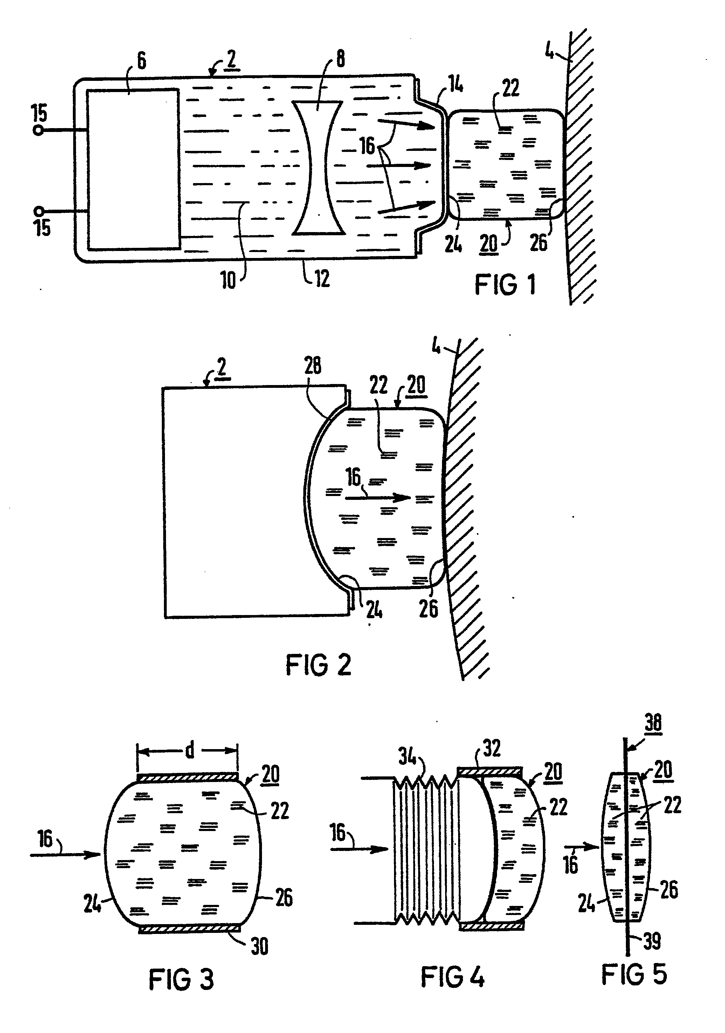

Dabei handelt es sich bei den in den Fig. 2 bis 4 und 7 dargestellten Gegenständen nicht um Ausführungsformen der Erfindung, sondern um Beispiele, die das Verständnis der Erfindung erleichtern sollen.

In Fig. 1 ist schematisch eine Stoßwellen-Therapieeinrichtung 2, und zwar speziell eine Zertrümmerungseinrichtung für Konkremente, wie z.B. Nierensteine, im Körper eines Patienten 4, dargestellt. Die gezeigte Stoßwellen-Therapieeinrichtung 2 ist an sich bekannt. Sie enthält eine Stoßwellenquelle 6, eine Linse 8 und ein Koppelmedium 10, wie z.B. Wasser. Diese Elemente 6, 8 und 10 sind in einem Gehäuse 12 untergebracht, das ausgangsseitig mit einer Membran 14 abgeschlossen ist. Die Membran 14 dient als Stoßwellen-Abstrahlfläche. Bei der Stoßwellenquelle 6 kann es sich um eine elektromagnetische Einrichtung, wie z.B. das sogenannte Stoßwellenrohr, oder aber auch um eine Piezokeramik handeln. Die elektrischen Anschlüsse für die Stoßwellenquelle 6 sind mit 15 bezeichnet. Die Hauptrichtung der von der Linse 8 fokussierten Stoßwellen ist mit einem Pfeil 16 angegeben.

Zwischen der Austritts-Membran 14 und dem zu behandelnden Patienten 4 ist ein Ankoppelkörper 20 angeordnet. Dieser Ankoppelkörper 20 dient also zur Übertragung der Stoßwellen auf dem Wege von der Stoßwellenquelle 6 einerseits und dem Patienten 4 andererseits. Der Ankoppelkörper 20 ist aus einem elastischen, formstabilen und allseitig feuchten Material 22, also auch mit feuchten Außenflächen 24, 26, gebildet. Er hat hier eine im wesentlichen zylindrische Gestalt.

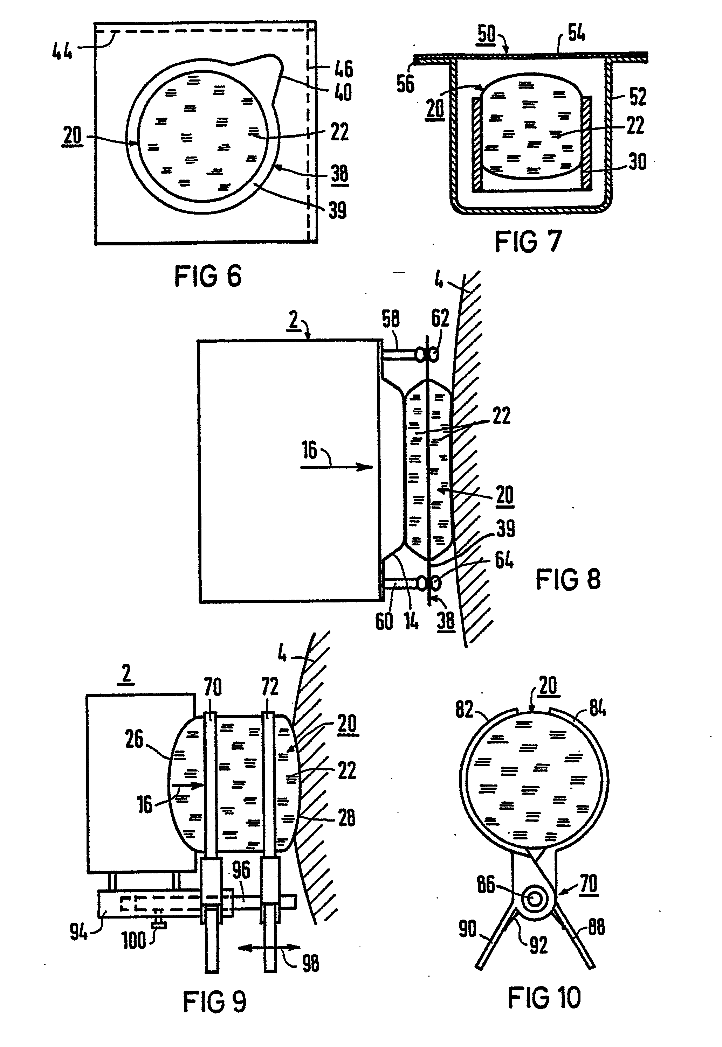

Bei dem Material 22 handelt es sich bevorzugt um ein Hydro-Gel wie ein Polyacrylamid-Gel. Ein solches Hydro-Gel ist viskoelastisch und schmiegt sich leicht mit seiner vorderen und hinteren feuchten Grenzfläche 24 bzw. 26 an die Membran 14 bzw. an den Patienten 4 an. Bei diesem Material handelt es sich also um keine Paste, die leicht verformbar und weich ist und nach der Benutzung sowohl an der Membran 14 als auch am Patienten 4 kleben bleibt. Die Matrix eines Hydro-Gels ist Wasser. Impedanz und Schallgeschwindigkeit des Ankoppelkörpers 20 lassen sich durch mehr oder minder große Beigabe von Wasser bei der Herstellung einstellen. Wie dargestellt, ist das Material 22 recht formstabil, so daß es prinzipiell ohne eine äußere feste Fassung (wie später in Fig. 3 und 4 gezeigt) einsetzbar ist. Es ist aber mit einer Einlage (wie später anhand der Figuren 5 ff. näher erläutert) versehen. Das Material 22 des Ankoppelkörpers 20 ist so vernetzt, daß es im wesentlichen homogen ist.

In Fig. 2 ist eine weitere Form einer Stoßwellen-Therapieeinrichtung 2 dargestellt. Diese Stoßwellen-Therapieeinrichtung 2 ist mit einer fokussierenden Stoßwellenquelle ausgerüstet. Dabei kann es sich entweder um einen piezokeramischen Körper mit einer kalottenförmigen Abstrahlfläche oder aber – wie dargestellt – um ein Stoßwellenrohr mit einer konkaven Spule und einem kalottenförmigen Abstrahlglied in Form einer Membran handeln. Letztere Ausführungsform eines Stoßwellenrohres mit konkaver Abstrahlfläche 28 ist beispielsweise in dem DE-GM 8 413 031 gezeigt und beschrieben.

Zwischen der Abstrahlfläche 28 und dem Patienten 4 ist wiederum ein Ankoppelkörper 20 aus einem elastischen, formstabilen Material 22, wieder mit feuchten Außenflächen 24, 26 gehaltert. In dieser Ausführungsform erfüllt der Ankoppelkörper 20, der wiederum bevorzugt aus einem Hydro-Gel besteht, eine doppelte Funktion: Zum einen dient er als Vorlaufstrecke, und zum anderen wird er infolge seiner feuchten Außenflächen 24, 26 als Ankoppelmedium benutzt.

In Fig. 3 ist ein Ankoppelkörper 20 gezeigt, der im wesentlichen zylindrisch geformt ist und an seinem Rand eingefaßt ist. Zur Einfassung ist ein flexibles Schlauchstück 30, beispielsweise ein Silikon-Schlauchstück, vorgesehen. Dieses hat eine dem Anwendungsfall und dem Ankoppelkörper 20 angepaßte Dicke d. Für Routineuntersuchungen können verschiedene Ankoppelkörper 20 mit unterschiedlichen Dicken d auf Lager gehalten werden. Wie dargestellt, sind die beiden endseitigen Ankoppelflächen 24, 26 konvex ausgebildet. Sie können sich beim Einsatz zwischen Stoßwellen-Austrittsfläche und Patient mehr oder weniger stark verformen. Als Material 22 wird auch hier wieder ein Hydro-Gel verwendet. Dieses zeichnet sich durch eine geringe Dämpfung für die Stoßwellen aus. Die Impedanz und die Schallgeschwindigkeit dieses Mediums können dem Körpergewebe angepaßt werden. Als Vorteil ist zu verzeichnen, daß sowohl das Schlauchstück 30 als auch das Material 22 für Röntgenstrahlung durchlässig sind, was eine Ortung des Konkrements bei aufgesetztem Ankoppelkörper 20 ermöglicht.

In der Ausführungsform nach Fig. 4 ist zur Einfassung des Ankoppelkörpers 20 ein Ring 32 vorgesehen. Dieser Ring 32 ist aus einem halbharten Material, das röntgenstrahlendurchlässig ist, gefertigt. Er ist auf ein entsprechend geformtes Gegenstück 34, das an die Stoßwellenquelle (nicht gezeigt) angeschlossen ist, aufschiebbar. Bei diesem Gegenstück 34 kann es sich insbesondere um einen Ankoppelbalg bekannter Ausgestaltung handeln.

Ein solcher Ankoppelbalg ist aufblasbar, um den Fokus der Zertrümmerungseinrichtung bei Feinregulierung einzustellen. Der Ring 32 und das Material 22 können in verschiedenen Dicken bereitgehalten werden; auf diese Weise läßt sich ein Ausgleich für verschiedene Dicken der untersuchten Patienten herbeiführen. Abweichend davon kann nur das scheibenförmig ausgebildete Material 22 in verschiedenen Dicken bereitgehalten werden, um es im Ring 32 je nach Patientendicke auszuwechseln.

In Fig. 5 ist ein Ankoppelkörper 20 gezeigt, der als dünne Scheibe geformt und mit einer ebenen Einlage 38 versehen ist. Bei dieser Einlage 38 handelt es sich um ein großmaschiges Gewebe (Gaze) aus einem röntgenstrahlendurchlässigen Material, z.B. aus einem Textilgewebe. Die Einarbeitung dieses Gewebes dient einer Erhöhung der mechanischen Festigkeit und einer Erleichterung der Handhabbarkeit des Ankoppelkörpers 20. Die Gewebeeinlage 38 kann durch die Mitte der Scheibe und sollte senkrecht zur Hauptstrahlrichtung 16 verlaufen. Es gibt aber auch Anwendungsfälle, wo die Einbettung der Gewebeeinlage 38 in der Nähe einer der beiden Ankoppelflächen 24, 26 vorteilhafter ist. Wie in Fig. 5 dargestellt, steht die Einlage 38 allseitig über das elastische, formstabile Material 22 randseitig über. Sie kann hier angefaßt werden. Der Einlagerand 39 dient hier als Mittel zur Erhöhung der Handhabbarkeit, z.B. bei einer Justierung.

In Fig. 6 ist gezeigt, daß die zwischengelegte Einlage 38 aus Gewebe mit einer Anfaßlasche 40 versehen sein kann. Der gesamte Ankoppelkörper 20 ist hier in einer Verpackung 42 untergebracht. Bei dieser Verpackung 42 kann es sich insbesondere um eine luftdicht geschlossene Klarsichthülle rechteckigen Formats handeln. Die Hülle kann aus einem Kunststoffbeutel, der z.B. aus Polyäthylen besteht, mit einer dünnen Metallisierung bestehen. Dieser Kunststoffbeutel ist luftdicht verschweißt und bildet somit einen Behälter. Auf diese Weise wird der Ankoppelkörper 20 gegen Austrocknen geschützt. Er läßt sich in der Verpackung 42 über längere Zeit transportieren und lagern. Erst unmittelbar vor Gebrauch wird der Ankoppelkörper 20 aus der Verpackung 42 herausgenommen. Das Herausnehmen wird durch Aufreißnähte 44 und 46 erleichtert.

Nach Fig. 7 kann das in einem Schlauchstück 30 randseitig eingefaßte Material 22 auch in einem topfförmigen Behälter 50, der luftdicht verschlossen ist, aufbewahrt werden. Dieser Behälter 50 besteht aus einem Töpfchen 52 mit umgebogenem Rand, auf dem eine durchsichtige Folie 54 verschweißt ist. Anstelle einer durchsichtigen Folie 54 kann auch eine kunststoffbeschichtete Metallfolie verwendet werden. Die Folie 54 ist mit einer Aufreißlasche 56 und/oder Aufreißnaht versehen. Die Konstruktion des Behälters 50 ist ähnlich der eines Joghurtbechers.

Die Ausführungsform nach Fig. 8 ist ähnlich derjenigen von Fig. 1. Hier ist gezeigt, daß zwischen der Stoßwellen-Austrittsfläche in Form der Membran 14 und dem Patienten 4 ein gelartiger Ankoppelkörper 20 mit einer Einlage 38 aus einem Gewebe festgehalten ist. Der überstehende Teil der eingearbeiteten Einlage 38 dient zur Befestigung des Ankoppelkörpers 20 an der Stoßwellenquelle der Stoßwellen-Therapieeinrichtung 2. Diese Befestigung ist durch zwei Haltestifte 58, 60 und in diese eingreifende Befestigungsknöpfe 62, 64 angedeutet. Prinzipiell kann auch jede andere Art der Befestigung gewählt werden.

In den Fig. 9 und 10 ist gezeigt, daß zur Befestigung des Ankoppelkörpers 20 an der Stoßwellen-Therapieeinrichtung 2 oder der Stoßwellenquelle zwei Klemmzangen 70, 72 vorgesehen sein können. Bei gewissen Anwendungsfällen wird man mit einer einzigen breiten Klemmzange 70 auskommen können. Die zweite Klemmzange 72 dient zur Anpassung an unterschiedliche Dicken des Ankoppelkörpers 20. Die Einlage 38 ist in den Figuren 9 und 10 aus Gründen der besseren Übersicht weggelassen.

Wie aus Fig. 10 hervorgeht, sind die beiden Klemmzangen 70, 72 ähnlich einer Wäscheklammer geformt. Sie umfassen im wesentlichen halbkreisförmige Einklemmschenkel 82, 84, die über eine Drehachse 86 mit Handhabungsschenkeln 88 bzw. 90 verbunden sind. Zwischen den beiden Handhabungsschenkeln 88, 90 liegt eine Spreizfeder 92, die im eingespannten Zustand den Einfaßdruck auf den Ankoppelkörper 20 ausübt. Zum Herausnehmen des Ankoppelkörpers 20 brauchen die beiden Handhabungsschenkel 88, 90 nur aufeinanderzu gedrückt zu werden.

Aus Fig. 9 ist deutlich, daß die erste Klemmzange 70 an einer Halterung 94 befestigt ist, die wiederum an der Stoßwellen-Therapieeinrichtung 2 festgemacht ist. Diese Halterung 94 kann im wesentlichen ein Rohr umfassen, in dem eine Stange 96 in Längsrichtung verschiebbar ist. An dieser Stange 96 ist die zweite Klemmzange 94 befestigt. Die Verschiebbarkeit in Längsrichtung ist durch einen Doppelpfeil 98 angedeutet. Um die zweite Klemmzange 72 und den Stab 96 in einer vorgegebenen Position zu arretieren, ist an der Halterung 94 eine Klemmeinrichtung 100, z.B. eine Klemmschraube, vorgesehen. Diese verhindert nach Einstellung eine Verschiebung des Stabes 96 in Richtung des Doppelpfeils 98, so daß der Ankoppelkörper 20 wirksam vor der konkaven Abstrahlfläche (z. B. in Form einer Membran) fixiert ist. Mittels der beiden Klemmzangen 70, 72 kann der Ankoppelkörper 20 aus einem elastischen, formstabilen Material mit feuchten Außenflächen 24, 26 leicht und rasch gegen einen anderen unterschiedlicher Dicke ausgewechselt werden.

Abschließend sei noch erwähnt, daß die dargestellten Mittel auch auf dem Gebiet der Ultraschall-Diagnose mit Ultraschall-Applikator anwendbar sind.

## Patentansprüche

1. Ankoppelkörper für die Übertragung von Stoßwellen von einer Stoßwellenquelle zu einem zu behandelnden Patienten, welcher Ankoppelkörper aus einem elastischen, formstabilen Material (22) mit feuchten Außenflächen (24, 26) gebildet und mit einer seine mechanische Festigkeit erhöhenden Einlage (38) versehen ist, wobei die Einlage (38) über das elastische, formstabile Material (22) übersteht und am überstehenden Teil Mittel (39, 40) zum Anfassen aufweist.

2. Ankoppelkörper nach Anspruch 1, dadurch gekennzeichnet, daß das Material (22) ein Hydro-Gel ist.

3. Ankoppelkörper nach Anspruch 2, dadurch gekennzeichnet, daß das Material (22) ein Polyacrylamid-Gel ist.

4. Ankoppelkörper nach einem der Ansprüche 1 bis 3, dadurch gekennzeichnet, daß das Material (22) im wesentlichen homogen ist.

5. Ankoppelkörper nach einem der Ansprüche 1 bis 4, dadurch gekennzeichnet, daß er im wesentlichen zylindrisch geformt ist.

6. Ankoppelkörper nach einem der Ansprüche 1 bis 5, dadurch gekennzeichnet, daß das elastische, formstabile Material (22) am Rand eingefaßt ist (Fig. 9).

7. Ankoppelkörper nach einem der Ansprüche 1 bis 6, dadurch gekennzeichnet, daß die Einlage (38) mit einer Anfaßlasche (40) versehen ist (Fig. 6).

8. Ankoppelkörper nach einem der Ansprüche 1 bis 7, dadurch gekennzeichnet, daß er in einem luftdicht verschlossenen Behälter (42; 50) untergebracht ist (Fig. 6).

9. Ankoppelkörper nach einem der Ansprüche 1 bis 8, dadurch gekennzeichnet, daß die Einlage (38) zur Befestigung an der Stoßwellenquelle (2) vorgesehen ist (Fig. 8).

10. Ankoppelkörper nach einem der Ansprüche 1 bis 9, dadurch gekennzeichnet, daß zu seiner Befestigung an der Stoßwellenquelle (2) mindestens eine Klemmzange (70, 72) vorgesehen ist (Fig. 9, 10).

## Claims

1. A coupling member for transmission of shock waves from a source of shock waves to a patient to be treated, said coupling member being made of an elastic, shape-retaining material (22) with moist outer surfaces (24, 26) and being provided with an insert (38) that increases its mechanical strength, said insert (38) projecting beyond the elastic, shape-retaining material (22) and having grasping means (39, 40) on the projecting part.

2. A coupling member according to claim 1, characterised in that the material (22) is a hydrogel.

3. A coupling member according to claim 2, characterised in that the material (22) is a polyacrylamide gel.

4. A coupling member according to any one of claims 1 to 3, characterised in that the material (22) is substantially homogeneous.

5. A coupling member according to any one of claims 1 to 4, characterised in that its shape is substantially cylindrical.

6. A coupling member according to any one of claims 1 to 5, characterised in that the elastic, shape-retaining material (22) is framed at its edge (Fig. 9).

7. A coupling member according to any one of claims 1 to 6, characterised in that the insert (38) is provided with a grasping tongue (40) (Fig. 6).

8. A coupling member according to any one of claims 1 to 7, characterised in that it is housed in an airtight sealed container (42; 50) (Fig. 6).

9. A coupling member according to any one of claims 1 to 8, characterised in that the insert (38) is intended for fastening to the source of shock waves (Fig. 8).

10. A coupling member according to any one of claims 1 to 9, characterised in that at least one clamping claw (70, 72) is provided for fastening to the source of shock waves (2) (Fig. 9, 10).

## Revendications

1. Corps de couplage pour la transmission d'ondes de choc depuis une source d'ondes de choc à un patient à traiter, lequel corps de couplage est réalisé en un matériau élastique de forme stable (22) possédant des surfaces extérieures humides (24, 26) et possède un insert (38) augmentant sa résistance mécanique, l'insert (38) faisant saillie par rapport au matériau élastique de forme stable (22) et possédant, sur sa partie saillante, des moyens de préhension (39, 40).

2. Corps de couplage suivant la revendication 1, caractérisé par le fait que le matériau (22) est un hydrogel.

3. Corps de couplage suivant la revendication 2, caractérisé par le fait que le matériau (22) est un gel de polyacrylamide.

4. Corps de couplage suivant l'une des revendications 1 à 3, caractérisé par le fait que le matériau (22) est sensiblement homogène.

5. Corps de couplage suivant l'une des revendica tions 1 à 4, caractérisé par le fait qu'il possède sensiblement une forme cylindrique.

6. Corps de couplage suivant l'une des revendications 1 à 5, caractérisé par le fait que le matériau élastique de forme stable (22) est serti sur son bord (figure 9).

7. Corps de couplage suivant l'une des revendications 1 à 6, caractérisé par le fait que l'insert (38) comporte une languette de préhension (figure 6).

8. Corps de couplage suivant l'une des revendications 1 à 7, caractérisé par le fait qu'il est logé dans un récipient (42; 50) fermé de façon étanche à l'air (figure 6).

9. Corps de couplage suivant l'une des revendications 1 à 8, caractérisé par le fait que l'insert (38) est destiné à être fixé sur la source d'ondes de choc (2) (figure 8).

10. Corps de couplage suivant l'une des revendications 1 à 9, caractérisé par le fait que, pour sa fixation sur la source d'ondes de choc (2), il comporte au moins une pince de serrage (70, 72) (figures 9, 10).

FIG 1

FIG 2

FIG 3

FIG 4

FIG 5

FIG 6

FIG 7

FIG 8

FIG 9

FIG 10